(19) Europäisches Patentamt
European Patent Office
Office européen des brevets

(11) **EP 2 918 338 A1**

(12) **EUROPÄISCHE PATENTANMELDUNG**

(43) Veröffentlichungstag:
**16.09.2015 Patentblatt 2015/38**

(51) Int Cl.:
***B01J 23/02*** *(2006.01)*      ***C07C 1/20*** *(2006.01)*
***C07C 11/167*** *(2006.01)*      ***B01J 37/02*** *(2006.01)*

(21) Anmeldenummer: **14159488.7**

(22) Anmeldetag: **13.03.2014**

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**

(71) Anmelder: **LANXESS Deutschland GmbH
50569 Köln (DE)**

(72) Erfinder:
• **Haufe, Rommy, Dr.
57439 Attendorn (DE)**
• **Reschetilowski, Wladimir, Prof. Prof. Dr.
01445 Radebeaul (DE)**
• **Thomas, Axel
01309 Dresden (DE)**
• **Hauser, Matthias
01689 Niederau (DE)**

(54) **Verfahren zur Herstellung von 1,3-Butadien**

(57) Die vorliegende Erfindung betrifft Katalysatoren für die 1,3-Butadien Synthese ausgehend von Ethanol sowie ein Verfahren zur Herstellung von 1,3-Butadien ausgehend von Ethanol unter Einsatz dieser Katalysatoren auf Basis von Sauerstoffverbindungen des Magnesiums, des Calciums und des Siliciums.

**EP 2 918 338 A1**

## Beschreibung

Die vorliegende Erfindung betrifft Katalysatoren für die 1,3-Butadien Synthese ausgehend von Ethanol sowie ein Verfahren zur Herstellung von 1,3-Butadien ausgehend von Ethanol unter Einsatz dieser Katalysatoren auf Basis von Sauerstoffverbindungen des Magnesiums, des Calciums und des Siliciums.

Die Herstellung von 1,3-Butadien aus Ethanol in Gegenwart anorganischer Katalysatoren hat insbesondere vor dem Hintergrund der Möglichkeit, Ethanol aus nicht-fossilen Quellen zu gewinnen, in den vergangenen Jahren wieder besonderes Interesse geweckt.

## Stand der Technik

Basierend auf den Arbeiten von Lebedev (GB 331482) wurde eine Vielzahl von Katalysatorsystemen entwickelt. Insbesondere solche, die Sauerstoffverbindungen des Magnesiums und Siliciums sowie gegebenenfalls Dotierungen mit anderen Metalloxiden wie Übergangsmetalloxiden aufweisen, haben sich als relativ selektiv erwiesen.

H. Niiyama et al beschreibt im Bulletin of the Chemical Society of Japan, Vol. 45, 655-659 (1972) $SiO_2$-MgO Katalysatoren mit einem Gehalt an MgO von 75mol% und 85 mol%. Erzielt wird gemäß Beispiel 1 in Tabelle 1 bei der Synthese von Butadien aus Ethanol eine Umsetzungsgrad (Conversion to butadiene) von lediglich 0,11.

In WO 2012/015340 A1 werden schließlich Katalysatoren für die Synthese von Butadien aus Ethanol auf Basis von Magnesiumoxid und Siliciumdioxid beschrieben, die Selektivitäten von 40 % aufweisen.

Allen bislang bekannten Verfahren ist gemeinsam, dass hohe 1,3-Butadien Selektivitäten bei gleichzeitig hoher Katalysatorbeladung nicht in kommerziell attraktivem Ausmaß erreicht werden können. Es bestand daher die Aufgabe Katalysatoren und ein Verfahren zu ihrer Verwendung bereitzustellen, wodurch die Nachteile des Standes der Technik überwunden werden und insbesondere bei niedriger Beladung eine hohe Selektivität bei der Bildung von 1,3-Butadien aus Ethanol gegeben ist.

Es wurde nun ein Katalysator auf Basis wenigstens einer Sauerstoffverbindung des Magnesiums, Calciumoxid und wenigstens einer Sauerstoffverbindung des Siliciums gefunden, der sich im Hinblick auf die Synthese von 1,3-Butadien aus Ethanol durch eine besonders hohe Selektivität auszeichnet.

**Gegenstand der vorliegenden Erfindung** ist ein Katalysator enthaltend

(I) wenigstens eine Sauerstoffverbindung des Magnesiums und wenigstens eine Sauerstoffverbindung des Siliciums und

(II) Calciumoxid.

Es sei an dieser Stelle angemerkt, dass der Rahmen der Erfindung alle beliebigen und möglichen Kombinationen der oben stehenden und im Folgenden aufgeführten, allgemeinen oder in Vorzugsbereichen genannten Komponenten, Wertebereiche bzw. Verfahrensparameter umfasst.

Bevorzugt betrifft die vorliegende Erfindung einen Katalysator enthaltend

(I) 90 bis 99,9 Mol.-%, bevorzugt 92 bis 99,9 Mol.-%, besonders bevorzugt 95 bis 99,9 Mol.-% wenigstens einer Sauerstoffverbindung des Magnesiums und wenigstens einer Sauerstoffverbindung des Siliciums und

(II) 0,1 bis 10 Mol.-%, bevorzugt 0,1 bis 8 Mol.-%, besonders bevorzugt 0,1 bis 5 Mol.-% Calciumoxid, mit der Maßgabe, dass die Summe aller Molprozente stets 100 ergibt.

Besonders bevorzugt betrifft die vorliegende Erfindung einen Katalysator bei dem in der Komponente (I) das molare Verhältnis von Magnesium zu Silicium im Bereich von 3,5:1 bis 15:1, vorzugsweise im Bereich von 4:1 bis 15 :1 und besonders bevorzugt im Bereich von 5,5:1 bis 12,5:1 liegt.

Unter **Sauerstoffverbindungen des Magnesiums** sind solche Verbindungen zu verstehen, in denen Sauerstoff entweder als Oxid-Ion, als Hydroxidion, als Carbonation oder kovalent an Magnesium gebunden vorliegt.

Bevorzugte Sauerstoffverbindungen des Magnesiums sind Magnesiumhydroxid, Magnesiumcarbonat und Magnesiumoxid, wobei Magnesiumoxid und Magnesiumhydroxid besonders bevorzugt sind. Magnesiumoxid ist insbesondere bevorzugt und ganz besonders bevorzugt ist kristallines Magnesiumoxid.

Unter **Sauerstoffverbindungen des Siliciums** sind solche Verbindungen zu verstehen, in denen Sauerstoff als Oxid-Ion, Hydroxidion oder kovalent an Silicium gebunden vorliegt.

Bevorzugte Sauerstoffverbindungen des Siliciums sind alle kristallinen oder nicht-kristallinen Modifikationen bzw. Erscheinungsformen des Siliciumdioxids und Kieselsäuren der Formel $H_{2n+2}Si_nO_{3n+1}$, wobei n eine natürliche Zahl ist.

Besonders bevorzugt sind pyrogene Kieselsäuren, wobei unter pyrogenen Kieselsäuren amorphe Siliciumdioxid-Pulver zu verstehen sind, die eine spezifische Oberfläche von 50 bis 600 $m^2$/g aufweisen. In einer weiteren Ausführungsform weisen die Sauerstoffverbindungen des Siliciums und insbesondere die kristallinen oder nicht-kristallinen Modifikationen bzw. Erscheinungsformen des Siliciumdioxids eine spezifische Oberfläche im Bereich von 150 bis 400 $m^2$/g, bevorzugt im Bereich von 150 bis 380 $m^2$/g, besonders bevorzugt im Bereich von 230 bis 380 $m^2$/g auf.

In einer weiteren Ausführungsform weisen die Sauerstoffverbindungen des Siliciums und insbesondere die kristallinen oder nicht-kristallinen Modifikationen bzw. Erscheinungsformen des Siliciumdioxids ein Porenvolumen von 0,2 bis 1,2 $cm^3$/g vorzugsweise 0,3 bis 1,1 $cm^3$/g gemessen durch Quecksilberintrusion nach DIN 66133 [Hg] auf.

Spezifische Beispiele für bevorzugte Sauerstoffverbindungen des Siliciums sind pyrogene Kieselsäuren des Typs HDK® N20 der Fa. Wacker, Silicas des Typs Norpro SS 6*137 und SS 6*138 der Fa. Saint Gobain, oder pyrogene Kieselsäuren vom Aerosil® Typ, insbesondere Aerosil® 380, der Evonik Industries AG.

Soweit nicht anders angegeben, werden die in dieser Anmeldung angegebenen spezifischen Oberflächen bestimmt nach ISO 9277.

Weiterhin werden unter dem Begriff "Sauerstoffverbindungen des Magnesiums und Sauerstoffverbindungen des Siliciums" auch Magnesium enthaltende Silikate subsumiert.

Dem Fachmann ist dabei klar, dass der Bindungscharakter von Magnesium-Sauerstoff-Bindungen nach dem Pauling-Modell der Elektronegativitätsdifferenz nicht ausschließlich aber überwiegend ionisch ist, während Silicium-Sauerstoff-Bindungen nicht ausschließlich aber eher kovalenten Charakter besitzen. Die oben genannten Definitionen schließen diese Mischformen mit ein.

Bevorzugte Magnesium enthaltende Silikate sind **Magnesiumsilikate,** insbesondere $Mg_3Si_4O_{10}(OH)_2$, bekannt auch als Talkum oder Talk, und $Mg_8[(OH)_2|Si_6O_{15}]_2 \cdot (4+8)H_2O$ (Sepiolith).

In einer bevorzugten Ausführungsform enthalten die erfindungsgemäßen Katalysatoren als Sauerstoffverbindungen des Siliciums pyrogene Kieselsäure sowie als Sauerstoffverbindung des Magnesiums Magnesiumoxid.

Als Calciumoxid [CAS No. 1305-78-8] wird üblicherweise pulverförmiges Calciumoxid eingesetzt, das durch thermisches Zersetzen von Calciumhydroxid, welches bei 550 °C unter Atmosphärendruck in Calciumoxid und Wasser zerfällt, hergestellt wird. Das unter diesen Bedingungen entstandene Calciumoxid ist wenig kristallin, also gut reaktionsfähig.

Die Zubereitung des Katalysators für seine weitere Nutzung erfolgt durch Mischen der als Edukte einzusetzenden Komponenten (I) und (II). Der erfindungsgemäße Katalysator kann entweder ausschließlich aus den Komponenten (I) und (II) bestehen, oder aber zusätzlich zu den Komponenten (I) und (II) noch weitere Komponenten enthalten. In diesem Fall sind die Komponenten (I) und (II) im Rahmen der angegebenen Mengenbereiche so zu variieren, dass die Summe aller Gewichtsprozente stets 100 ergibt.

In einer weiteren Ausführungsform enthalten die erfindungsgemäßen Katalysatoren von 70 bis 100 Gew.-% Sauerstoffverbindungen des Magnesiums und des Siliciums sowie Calciumoxid, bevorzugt 90 bis 100 Gew.-%, besonders bevorzugt 98 bis 100 Gew.-%. Weitere Komponenten können Übergangsmetallverbindungen, Inertstoffe oder Alkalimetallverbindungen sein.

### Übergangsmetallverbindungen

In einer weiteren Ausführungsform enthalten die erfindungsgemäßen Katalysatoren als weitere Komponente zumindest eine Übergangsmetallverbindung, wobei die Übergangsmetallverbindungen vorzugsweise ausgewählt sind aus Sauerstoff-Verbindungen der Elemente Titan, Vanadium, Molybdän, Wolfram, Mangan, Kupfer, Nickel, Zink oder Chrom.

Bevorzugte Sauerstoff-Verbindung des Titan ist Titan(IV)oxid.

Bevorzugte Sauerstoffverbindung des Vanadium ist Vanadium(V)oxid.

Bevorzugte Sauerstoffverbindung des Mangan ist Mangan(II)oxid.

Bevorzugte Sauerstoffverbindung des Kupfer ist Kupfer(II)oxid.

Bevorzugte Sauerstoffverbindung des Nickel ist Nickel(II)oxid.

Bevorzugte Sauerstoffverbindung des Zink ist Zink(II)oxid.

Bevorzugte Sauerstoffverbindung des Chrom ist Chrom(III)oxid.

Bevorzugt sind als Übergangsmetallverbindung Sauerstoff-Verbindungen der Elemente Titan, Mangan, Kupfer, Nickel, Zink oder Chrom.

Besonders bevorzugt sind Sauerstoff-Verbindungen des Zinks oder Nickels, ganz besonders bevorzugt Zink(II)oxid oder Nickel(II)oxid, insbesondere bevorzugt ist Zink(II)oxid.

In einer Ausführungsform enthalten die erfindungsgemäßen Katalysatoren

(A) 70 bis 99,9 Gew.-% Sauerstoffverbindungen des Magnesiums und des Siliciums sowie Calciumoxid und

(B) 0,1 bis 30 Gew.-% mindestens einer Übergangsmetallverbindung, wobei die Summe aller Gewichtsprozente stets 100 ergibt.

Im Falle der Anwesenheit von Übergangsmetallverbindungen sind die Komponenten (A) und (B) im Rahmen der angegebenen Mengenbereiche so zu variieren, dass die Summe aller Gewichtsprozente stets 100 ergibt. Bevorzugt enthalten die Katalysatoren die Komponente (B) zu 0,1 bis 10 Gew.-%, besonders bevorzugt zu 0,1 bis 1 Gew.-%.

Im Falle der Anwesenheit von Übergangsmetallen beträgt das molare Verhältnis von Magnesium zu Übergangsmetall 15:1 bis 1000:1, vorzugsweise 50:1 bis 750:1, besonders bevorzugt von 75:1 bis 500:1 und ganz besonders bevorzugt von 80:1 bis 350:1.

Weitere Komponenten im Sinne der vorliegenden Erfindung sind bevorzugt Sauerstoffverbindungen anderer Metalle als Magnesium oder Calcium oder alternative oder zusätzliche **Inertstoffe,** die unter den Reaktionsbedingungen der 1,3-Butadiensynthese aus Ethanol nicht mit dem Edukt oder dem Produkt reagieren. Bevorzugte Inertstoffe sind Graphit, Siliciumcarbid oder Bornitrid.

In einer Ausführungsform enthalten die erfindungsgemäßen Katalysatoren 0,1 Gew.-% oder weniger an **Alkalimetallverbindungen** berechnet auf $M_2O$ wobei M ein Alkalimetall, bevorzugt Lithium, Natrium oder Kalium ist. Vorzugsweise enthalten die erfindungsgemäßen Katalysatoren weniger als 0,08 Gew.-% Alkalimetallverbindungen berechnet auf $M_2O$.

In einer Ausführungsform beträgt die **spezifische Oberfläche** der erfindungsgemäßen Katalysatoren 20 bis 500 $m^2$/g, vorzugsweise 20 bis 250 $m^2$/g und besonders bevorzugt 100 bis 250 $m^2$/g.

In einer weiteren Ausführungsform beträgt das **Porenvolumen** der erfindungsgemäßen Katalysatoren gemessen nach DIN 66 133 von 0,05 bis 0,4 $cm^3$/g, vorzugsweise 0,1 bis 0,4 $cm^3$/g und besonders bevorzugt 0,13 bis 0,34 $cm^3$/g.

Insbesondere betrifft die vorliegende Erfindung einen Katalysator enthaltend

(I) wenigstens MgO und wenigstens $SiO_2$ und

(II) CaO.

**Verfahren zur Herstellung des Katalysators**

Die Herstellung der erfindungsgemäßen Katalysatoren erfolgt bevorzugt in einem **Verfahren,** das zumindest folgende Schritte umfasst:

A) Herstellung eines Katalysatormaterials durch

ia) Fällung zumindest einer im Wesentlichen wasserlöslichen Magnesiumverbindung auf zumindest eine feste Sauerstoffverbindung des Siliciums und Calciumoxid, oder

ib) Fällung zumindest einer im Wesentlichen wasserlöslichen Calciumverbindung auf zumindest eine feste Sauerstoffverbindung des Siliciums und auf zumindest eine feste Sauerstoffverbindung des Magnesiums, oder

ic) Fällung zumindest einer nicht festen Siliciumverbindung auf zumindest eine feste Sauerstoffverbindung des Magnesiums und Calciumoxid, oder

id) Fällung zumindest einer nicht festen Siliciumverbindung und anschließend einer wasserlöslichen Calciumverbindung auf zumindest eine feste Sauerstoffverbindung des Magnesiums, oder

ie) Fällung zumindest einer nicht festen Siliciumverbindung und anschließend einer wasserlöslichen Magnesiumverbindung auf Calciumoxid, oder

if) Fällung zumindest einer löslichen Magnesiumverbindung und zumindest einer löslichen Calciumverbindung auf zumindest eine feste Sauerstoffverbindung des Siliciums,
jeweils in einem wässrigen Reaktionsmedium und Abtrennung des so erhaltenen, gefällten Katalysatormaterials vom wässrigen Reaktionsmedium,

ii) Mischen von Sauerstoffverbindungen des Magnesiums, Calciumoxid und Sauerstoffverbindungen des Siliciums als Katalysatormaterial,

B) gegebenenfalls Vermahlung des Katalysatormaterials,

C) gegebenenfalls Herstellung von Formkörpern enthaltend das gemäß Schritt A) und/oder B) erhaltene Katalysatormaterial,

D) Kalzinierung des Katalysatormaterials oder der Formkörper.

Gemäß Schritt A) wird ein Katalysatormaterial durch Fällung in einem wässrigen Reaktionsmedium gewonnen.

Der Begriff "wässriges Reaktionsmedium" bedeutet im Rahmen der Erfindung ein flüssiges, Wasser enthaltendes Medium, das vorzugsweise mindestens 80 Gew.-% Wasser enthält und besonders bevorzugt frei von organischen Lösungsmitteln ist, die nicht aus den eingesetzten Magnesium- oder Siliciumverbindungen und Calciumoxid stammen.

Der Begriff "Fällung" in einem wässrigen Reaktionsmedium umfasst die Konvertierung von im Wesentlichen wasserlöslichen Magnesiumverbindungen, von im Wesentlichen wasserlöslichen Calciumverbindungen oder nicht festen Siliciumverbindungen zu nicht löslichen Sauerstoffverbindungen des Magnesiums oder Magnesiumcarbonat, zu Calciumoxid oder Calciumcarbonat, bzw. nicht löslichen Sauerstoffverbindungen des Siliciums.

Nicht feste Siliciumverbindungen sind entweder wasserlösliche Siliciumverbindungen oder Siliciumverbindungen, die einen Schmelzpunkt von weniger als 20°C bei 1 bar aufweisen.

Im Wesentlichen wasserlösliche Magnesiumverbindungen und wasserlösliche Siliciumverbindungen sind solche, die bei 20°C und einem Druck von 1 bar eine Wasserlöslichkeit von mindestens 5,0 g/l aufweisen.

Im Wesentlichen wasserunlösliche Magnesium-, Calcium- und Siliciumverbindungen sind dementsprechend solche, die bei 20°C und einem Druck von 1 bar eine Wasserlöslichkeit von weniger als 5,0 g/l aufweisen.

Geeignete, im Wesentlichen wasserlösliche Magnesiumverbindungen sind bevorzugt Magnesiumnitrat, -sulfat, -chlorid, -bromid, -acetat, oder deren Hydrate oder Mischungen der vorgenannten Verbindungen.

Geeignete, im Wesentlichen wasserlösliche Calciumverbindungen sind bevorzugt Calciumnitrat, -chlorid, -bromid, -acetat, oder deren Hydrate oder Mischungen der vorgenannten Verbindungen

Wasserlösliche Siliciumverbindungen sind bevorzugt Wassergläser oder Siliciumverbindungen, die einen Schmelzpunkt von weniger als 20°C bei 1 bar aufweisen. Besonders bevorzugt sind Tetraethylorthosilicat, Tetramethylorthosilicat oder Tetraisopropylorthosilicat.

Für Schritt A) bevorzugt zu verwendende feste Sauerstoffverbindungen des Magnesiums und/oder Siliciums sind:

Magnesiumoxid, Magnesiumcarbonat und Magnesiumhydroxid, die oben genannten Sauerstoffverbindungen des Siliciums oder die oben genannten Magnesiumsilicate.

Die Fällung von Magnesiumverbindungen erfolgt bevorzugt derart, dass bei Raumtemperatur der pH-Wert des wässrigen Reaktionsmediums bei der Konvertierungstemperatur durch Zugabe von Basen auf über 9, vorzugsweise 9.6 bis 11.1 eingestellt wird.

Die Fällung von Calciumoxid oder Calciumcarbonat erfolgt bevorzugt derart, dass bei Raumtemperatur der pH-Wert des wässrigen Reaktionsmediums bei der Konvertierungstemperatur durch Zugabe von Basen auf über 12, vorzugsweise 12.4 bis 13.9 eingestellt wird.

Die **Fällung** von Siliciumverbindungen erfolgt bevorzugt derart, dass bei Raumtemperatur der pH-Wert des wässrigen Reaktionsmediums bei der Konvertierungstemperatur auf 2 bis 12 eingestellt wird.

Die Temperatur bei der Fällung von Magnesiumverbindungen, Calciumoxid/Calciumcarbonat und Siliciumverbindungen (Konvertierungstemperatur) liegt vorzugsweise im Bereich von 0 bis 100°C, besonders bevorzugt im Bereich von 20 bis 85°C und ganz besonders bevorzugt im Bereich von 50 bis 80°C.

Geeignete Basen sind vorzugsweise solche, die in Wasser einen $pK_B$-Wert bei Standardbedingungen von 5,0 oder weniger, vorzugsweise 4,0 oder weniger aufweisen. Bevorzugte Basen sind Alkalimetallcarbonate und -hydroxide, insbesondere Lithium-, Natrium- oder Kaliumcarbonat sowie Lithium-, Natrium oder Kaliumhydroxid.

Im Schritt A)ii) erfolgt die Abtrennung des gemäß der verschiedenen in A)i) erhaltenen, gefällten Katalysatormaterials vom wässrigen Reaktionsmedium.

Die **Abtrennung** erfolgt vorzugsweise durch Filtration, Zentrifugation oder Sedimentation und Dekantieren. Es sind aber auch andere dem Fachmann bekannte Fest-Flüssig-Trennoperationen durchführbar. Insbesondere erfolgt die Abtrennung durch Filtration.

Gemäß Schritt B) kann optional, aber bevorzugt die **Vermahlung** erfolgen.

Die Vermahlung kann in an sich bekannter Weise, vorzugsweise durch eine Kugelmühle oder einen Mixer, erfolgen. Typische Zeiträume liegen bevorzugt im Bereich von 5 min bis 72 Stunden, besonders bevorzugt im Bereich von 1 Stunde bis 24 Stunden.

Gemäß Schritt C) kann optional die **Herstellung von Formkörpern** erfolgen. Schritt C) kann auch nach Schritt D) erfolgen oder aber nach Schritt A) bzw. B).

Die Herstellung der Formkörper kann in an sich bekannter Weise, bevorzugt durch Tablettierung oder Extrudierung erfolgen. Prinzipiell sind aber auch andere, dem Fachmann bekannte Methoden möglich.

Im Schritt D) erfolgt die **Kalzinierung.**

Im Rahmen der Erfindung ist unter dem Begriff "Kalzinierung" eine thermische Behandlung des Katalysatormaterials oder der Formkörper bei einer Temperatur von 250 bis 1500°C, vorzugsweise 300 bis 1000°C zu verstehen.

Die Kalzinierung erfolgt vorzugsweise über einen Zeitraum im Bereich von 30 min bis 120 h Stunden, besonders bevorzugt über einen Zeitraum im Bereich von 2 h bis 12 h. Längere Kalzinierungen zeigen typischerweise keinen Effekt.

In einer Ausführungsform der Erfindung kann das Kalzinieren in dem Reaktor durchgeführt werden, der für das erfindungsgemäße Verfahren der Umsetzung von Ethanol zu 1,3-Butadien eingesetzt wird.

Beim Kalzinieren kann teilweise ein Gewichtsverlust beobachtet werden, der typischerweise auf Eliminierungsreaktionen von Wasser aus Hydroxy-Verbindungen wie Magnesiumhydroxid, Calciumhydroxid, Kieselsäuren oder Kohlendioxid aus Carbonaten zurückzuführen ist.

Sofern die erfindungsgemäßen Katalysatoren Übergangsmetallverbindungen, vorzugsweise ausgewählt aus Sauerstoff-Verbindungen der Elemente Titan, Vanadium, Molybdän, Wolfram, Mangan, Kupfer, Nickel, Zink oder Chrom enthalten, können diese beispielsweise im Schritt A) durch Co-Fällung von wasserlöslichen Übergangsmetallverbindungen mit den wasserlöslichen Magnesiumverbindungen und den wasserlöslichen Calciumverbindungen eingebracht werden. Typischerweise entstehen dabei die entsprechenden Oxide oder Hydroxide. Alternativ können die Übergangsmetalle aber auch direkt, vorzugsweise in Form von Oxiden, Hydroxiden, Nitraten oder Carbonaten im Schritt A) ii) der Mischung zugesetzt werden.

In einer Ausführungsform lassen sich die erfindungsgemäßen Katalysatoren wie folgt herstellen:

A) Herstellung eines Katalysatormaterials durch Vermischung von

- Kieselgelen oder pyrogener Kieselsäure oder Mischungen davon, vorzugsweise pyrogener Kieselsäure, mit

- Magnesiumhydroxid, Magnesiumoxid oder Magnesiumcarbonat, vorzugsweise Magnesiumoxid, und

- Calciumhydroxid, Calciumoxid oder Calciumcarbonat, vorzugsweise Calciumhydroxid oder Calciumoxid, insbesondere Calciumoxid und

- gegebenenfalls Übergangsmetallverbindungen, vorzugsweise Übergangsmetall -oxiden, -hydroxiden, -nitraten oder -carbonaten, vorzugsweise Oxide, Hydroxide, Nitrate oder Carbonate von Titan, Vanadium, Molybdän, Wolfram, Mangan, Kupfer, Nickel, Zink oder Chrom, besonders bevorzugt die Hydroxide und Oxide von Titan, Vanadium, Mangan, Kupfer, Nickel, Zink oder Chrom sowie Mangan, Zink-, Nickel und Kupfercarbonat,

B) Vermahlung des gemäß Schritt A) erhaltenen Katalysatormaterials,

C) gegebenenfalls Herstellung von Formkörpern enthaltend das gemäß Schritt A) und/oder B) erhaltene Katalysatormaterial,

D) Kalzinierung des Katalysatormaterials oder der Formkörper.

In einem Aspekt betrifft die Erfindung auch Katalysatoren erhältlich nach dem erfindungsgemäßen Verfahren.

**Verfahren zur Herstellung von 1,3-Butadien**

Die erfindungsgemäßen Katalysatoren eignen sich insbesondere zum Einsatz in einem Verfahren zur Herstellung von 1,3-Butadien aus Ethanol.

In einer Ausführungsform des erfindungsgemäßen Verfahrens erfolgt die Herstellung von 1,3-Butadien durch Inkontaktbringen eines Ethanol enthaltenden Gasstromes mit erfindungsgemäßem Katalysator in einem Festbettreaktor. Alternativ kann die Reaktion in einem Wirbelschichtreaktor durchgeführt werden. Eine weitere alternative Ausführungsform ist die Reaktionsführung in einem Zwei-Zonen-Wirbelschichtreaktor.

Das Ethanol im Ethanol enthaltenden Gasstrom hat vorzugsweise einen Partialdruck im Bereich von 0,01 bis 100 % bezogen auf den Gesamtdruck des Gasstromes, besonders bevorzugt im Bereich von 0,1 bis 100 %, in einer alternativen Ausführungsform im Bereich von 0,1 bis 10 %. Der Gasstrom kann weiterhin Inertgase enthalten oder in einer alternativen aber bevorzugten Ausführungsform auch nicht, wobei unter Inertgasen solche Gase zu verstehen sind, die bei den Reaktionsbedingungen nicht oder nicht messbar reagieren. Bevorzugte Inertgase sind Edelgase, insbesondere Argon sowie andere Gase, insbesondere Stickstoff. Wasser ist in diesem Reaktionssystem der Synthese von 1,3-Butadien aus Ethanol kein Inertgas.

Der Gasstrom kann in einer Ausführungsform zudem Wasser enthalten, wobei in diesem Fall der Wassergehalt im Gasstrom bezogen auf Ethanol vorzugsweise mehr als 0 bis 50 Gew.-%, besonders bevorzugt mehr als 0 bis 25 Gew.-%, ganz besonders bevorzugt mehr als 0 bis 10 Gew.-%, insbesondere bevorzugt 0,5 bis 5 Gew.-% beträgt, und in einer anderen Ausführungsform einer Menge, die dem Ethanol-Wasser Azeotrop bei den gewählten Reaktionsbedingungen entspricht.

In einer anderen Ausführungsform beträgt der Wassergehalt im Gasstrom bezogen auf Ethanol vorzugsweise 10.000 Gew.-ppm oder weniger, besonders bevorzugt 1.000 Gew.-ppm oder weniger.

Der Ethanol enthaltende Gasstrom kann vorzugsweise durch Kontaktieren, insbesondere Überströmen oder Einleiten eines Inertgasstromes über bzw. in Ethanol erfolgen, wobei über die Temperatur die Sättigung bzw. der Gehalt des Ethanols im Ethanol enthaltenden Gasstrom eingestellt werden kann. Alternativ ist simples Verdampfen von Ethanol möglich.

Die Temperatur beim Inkontaktbringen des Ethanol enthaltenden Gasstromes mit dem erfindungsgemäßen Katalysator liegt vorzugsweise im Bereich von 200°C bis 500°C, besonders bevorzugt im Bereich von 250°C bis 475°C, ganz besonders bevorzugt im Bereich von 325°C bis 475°C und insbesondere bevorzugt im Bereich von 350 bis 475°C.

Der Druck beim Inkontaktbringen des Ethanol enthaltenden Gasstromes mit dem erfindungsgemäßen Katalysator liegt vorzugsweise im Bereich von 1 bis 10 bar, besonders bevorzugt im Bereich von 1 bis 5,5 bar, insbesondere bevorzugt im Bereich des Umgebungsdrucks.

Das Verfahren zur Herstellung von 1,3-Butadien mit den erfindungsgemäßen Katalysatoren kann batchweise oder kontinuierlich betrieben werden. In einer bevorzugten Ausführungsform wird das Verfahren kontinuierlich betrieben.

Erfindungsgemäß wird ein Produktgasgemisch erhalten, das 1,3-Butadien, sowie gegebenenfalls nicht umgesetztes Ethanol und Nebenprodukte enthält. Bevorzugt werden als Nebenprodukte Ethylen und/oder Aceton gebildet.

Die Abtrennung von 1,3-Butadien aus dem Produktgasgemisch erfolgt bevorzugt durch Ausfrieren. Prinzipiell sind aber auch andere, dem Fachmann bekannte thermische oder selektiv adsorptive Trennverfahren durchführbar.

In einer Ausführungsform des erfindungsgemäßen Verfahrens wird die Katalysatorbeladung so gewählt, dass sie mehr als 0,25, vorzugsweise 0,5 bis 20,0, bevorzugt 1 bis 15,0, in einer anderen Ausführungsform 0,5 bis 10,0 und besonders bevorzugt 1,0 bis 5,0 g Ethanol / g Katalysator beträgt.

In einer weiteren Ausführungsform des erfindungsgemäßen Verfahrens werden Reaktionstemperatur, Druck, Beladung und Verweilzeit so gewählt, dass mehr als 40 Gew.-% des eingesetzten Ethanols umgesetzt werden, vorzugsweise mehr als 70 Gew.-% und besonders bevorzugt mehr als 80 Gew.-%.

Gegebenenfalls nicht umgesetztes Ethanol kann erneut in die Reaktion zurückgeführt werden.

Die Einsatzzeit der Katalysatoren, insbesondere in kontinuierlichen Verfahren beträgt typischerweise 5 Stunden oder mehr.

Die Reaktionsführung wird anhand der Beispiele näher erläutert.

In einer alternativen Ausführungsform können statt des Calciumoxids [CAS No. 1305-78-8] alternative Sauerstoffverbindungen des Calciums eingesetzt werden, worin Sauerstoff entweder als Oxid-Ion, Hydroxidion, Silikation oder kovalent an Calcium gebunden vorliegt. Bevorzugte alternative Sauerstoffverbindungen des Calciums ist Calciumhydroxid, bevorzugte Calciumsilikate werden durch die Summenformel $CaSiO_3$ charakterisiert. Besonders bevorzugt ist Wollastonit $Ca_3[Si_3O_9]$, [CAS No. 13983-17-0].

## Beispiele

## Allgemeine Vorschrift zur Katalysatorherstellung

Als Sauerstoffverbindung des Siliciums wurde pyrogene Kieselsäure des Typs HDK® N20 der Fa. Wacker, nachstehend HDK genannt, eingesetzt. Sie wies folgende Spezifikation auf.

| Eigenschaft | Einheit | Wert |
|---|---|---|
| spezifische Oberfläche | $[m^2/g]$ | 233 |
| Porenvolumen [Hg] | $[cm^3/g]$ | 0,31 |
| Partikelgrößenverteilung | 10 % aller Partikel (Anzahl) | 8 $\mu$m |
| | 50 % aller Partikel | 38 $\mu$m |
| | 90 % aller Partikel | 91 $\mu$m |
| Schmelzpunkt | [°C] | 1700 |
| Dichte (DIN 51757) | $[g/cm^3]$ | 2,2 |

(fortgesetzt)

| Eigenschaft | Einheit | Wert |
|---|---|---|
| Schüttdichte | [kg/m$^3$] | 20 - 130 |
| pH-Wert (DIN EN ISO 787-9) | [-] | 3,6 - 4,5 |

Als Sauerstoffverbindung des Magnesiums bzw. Calciums wurde MgO bzw. CaO eingesetzt, welche aus Mg(OH)$_2$ (Sigma Aldrich) und Ca(OH)$_2$ (Sigma Aldrich) durch folgende Vorbehandlung erhalten wurden:

Mg(OH)$_2$ wurde in eine Keramikschale gegeben und im Muffelofen erhitzt (Heizrate: 10K/min, Endtemperatur: 900°C, Haltezeit: 4h)

Ca(OH)$_2$ wurde in eine Keramikschale gegeben und im Muffelofen erhitzt (Heizrate: 10K/min, Endtemperatur: 750°C, Haltezeit: 6h)

**Herstellung der Katalysatoren**

Zunächst wurde sowohl von HDK, als auch von den thermisch vorbehandelten MgO- und CaO-Präkursoren mittels thermogravimetrischer Analyse der Restfeuchte-Gehalt ermittelt. Über den Reinheitsgrad, den Restfeuchte-Gehalt und die Molare Masse aller drei Komponenten wurde für die gewünschte molare Zusammensetzung die jeweils einzuwiegende Masse berechnet.

**Beispiel 1 (Vergleichsbeispiel)** binärer Mischoxidkatalysator MgO-HDK-85:15

Zur Herstellung des Katalysators gemäß Beispiel 1 (MgO-HDK-85:15) wurden 11,985 g (0,297 mol) MgO (Reinheit 95%, TGA-Rückstand 99,015%) und 3,015 g (0,050 mol) HDK (Reinheit 99%, TGA Rückstand 99,382%) in einem Becherglas eingewogen und anschließend wurde das Gemenge in einen 500 ml Rundkolben überführt. Unter Schwenken des Rundkolbens wurden ca. 200 ml entionisiertes Wasser zugegeben, so dass sich eine Suspension ohne erkennbaren Rückstand am Boden des Rundkolbens bildete. Dann wurde die Suspension am Rotationsverdampfer unter Vakuum zunächst für 30 min bei 70°C homogenisiert und anschließend bei 170 mbar bis zur Trockne eingeengt. Das Feststoffgemisch wurde nun für 16 h in einen auf 70 °C temperierten Trockenschrank gestellt und das getrocknete Gemenge anschließend in einem Ofen für 6h bei 700°C im Luftstrom (15 l/h) calciniert. Vor der katalytischen Austestung wurde die calcinierte Katalysatorprobe bei 15-20 bar verpresst und im Anschluss auf eine Fraktion zwischen 200 und 400 μm klassiert. Der so erhaltene Katalysator besaß eine Zusammensetzung von 85 mol-% MgO und 15 mol-% SiO$_2$ in Form von HDK.

**Beispiel 2)** erfindungsgemäßer ternärer Mischoxidkatalysator MgO-CaO-HDK-84,9:0,1:15

Abgewogen wurden 8,562g (0,2124mol) MgO (Reinheit:95%, TGA Rückstand: 97,185%), 0,014g (0,00025 mol) CaO (Reinheit: 95%, TGA Rückstand: 96,028%) und 2,103g (0,0350 mol) HDK (Reinheit: 99%, TGA-Rückstand: 100%) in einem Becherglas und anschließend das Gemenge in einen 500ml-Rundkolben überführt. Unter Schwenken des Rundkolbens wurden ca. 200 ml entionisiertes Wasser zugegeben, so dass sich eine Suspension ohne erkennbaren Rückstand am Boden des Rundkolbens bildete. Am Rotationsverdampfer wurde die Suspension unter Vakuum zunächst für

30 min bei 70 °C homogenisiert und anschließend bei 170 mbar bis zur Trockne eingeengt. Das Feststoffgemisch wurde nun für 16 h in einen auf 70 °C temperierten Trockenschrank gestellt. Das getrocknete Gemenge wurde anschließend in einem Ofen für 6 h bei 700 °C im Luftstrom (15 l/h) calciniert. Vor der katalytischen Austestung wurde die calcinierte Katalysatorprobe bei 15-20 bar verpresst und im Anschluss auf eine Fraktion zwischen 200 und 400 μm klassiert. Der so erhaltene Katalysator besaß eine Zusammensetzung von 84,9 mol-% MgO, 0,1 mol-% CaO und 15 mol-% SiO$_2$ in Form von H DK. (TGA = Thermogravimetrie).

Analog zu Beispiel 2) wurden die Beispiele 3) und 4) unter Verwendung der folgenden Mengen Magnesiumoxid, Calciumoxid und Siliziumdioxid (in Form von HDK) hergestellt:

**Beispiel 3)** 8,248 g (0,2046 mol) MgO, 0,338 g (0,0060 mol) CaO, 2,085 g (0,0347 mol) HDK. Dabei wurde ein Katalysator bestehend aus 82,5 mol-% MgO, 2,5 mol-% CaO und 15 mol-% SiO$_2$ in Form von HDK erhalten. (TGA-Rückstände MgO / CaO / HDK: 97,185 % / 96,028 % / 100 %)

**Beispiel 4)** 7,378 g (0,1830 mol) MgO, 0,650 g (0,0116 mol) CaO, 1,972 g (0,0328 mol) HDK. Dabei wurde ein Katalysator bestehend aus 80 mol-% MgO, 5 mol-% CaO und 15 mol-% $SiO_2$ in Form von HDK erhalten. (TGA-Rückstände MgO / CaO / HDK: 99,015 % / 93,763 % / 99,382 %)

**Allgemeine Vorschrift zur Durchführung der katalytischen Tests**

**Versuchsaufbau**

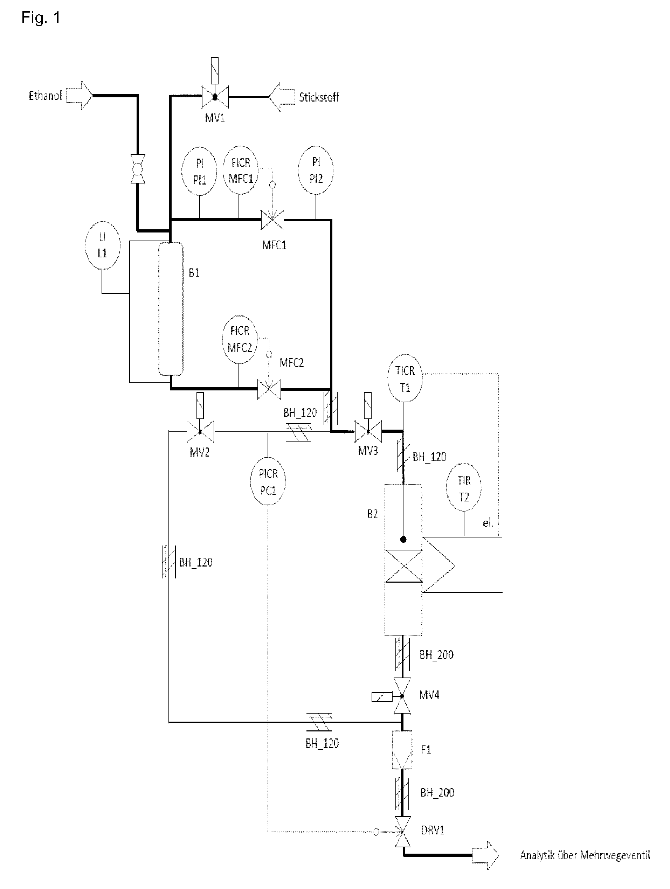

Die verwendete Laborversuchsanlage bestand aus 6 parallelen Einzelreaktoranlagen, welche über ein steuerbares Mehrwegeventil an die gaschromatographische Analytik angeschlossen waren. Der Versuchsaufbau eines Einzelreaktors ist in **Fig. 1** schematisch dargestellt.

Das Edukt (Ethanol mit 6 wt.-% Wasser) wurde in einem Vorratsgefäß **B1** unter Druck gelagert und über den Massedurchflussregler **MFC2** gefördert. Das inerte Trägergas Stickstoff wurde über den Massedurchflussregler **MFC1** eingebracht.

Durch die elektrische Heizung **BH_120** wurde das Edukt verdampft und gasförmig dem Reaktor **B2** zugeführt. Der Reaktor wurde elektrisch beheizt und über das Thermoelement **T1** geregelt.

Als Reaktor **B2** wurde ein Edelstahl-Rohrreaktor mit einem Innendurchmesser von 14 mm verwendet. Der Katalysator wurde mit Hilfe von Quarzwolle fixiert. Für die katalytischen Messungen wurden jeweils 500 mg Katalysator verwendet.

Der Reaktionsdruck in der Anlage wurde durch das Druckregelventil **DRV1** in Verbindung mit dem Druckmessumformer **PC1** bei 4,5 bar Überdruck konstant gehalten.

Der Produktstrom wurde über die elektrische Heizung der Leitungen **BH_200** gasförmig dem Mehrwegeventil zugeführt. Über dieses Ventil konnte einer Probenschleife automatisch das Produktgemisch eines Reaktors zugeführt werden. Durch die Temperatur und Druckregelung der Probenschleife wurde eine konstante Probenmenge für alle Messungen gewährleistet.

Vor jedem katalytischen Test erfolgte im Reaktor **B2** eine Kalzination der Probe über 5 Stunden bei 550 °C in Stickstoff (10 l/h). Die Analyse des Produktgemisches erfolgte bei 7 h, 10 h und 13 h TOS (= Dauer der Ethanolüberleitung - Time On Stream). Im Folgenden werden die Mittelwerte dieser 3 Messungen für jede Katalysatorprobe angegeben.

**Analytik**

Die Auswertung der erhaltenen Ergebnisse erfolgte über die Bildung der Kohlenstoffbilanz. Diese setzt die Analyse aller auftretenden Verbindungen voraus. Sichergestellt wurde dies durch eine gaschromatographische Analyse, welche mit folgenden Parametern durchgeführt wurde:

| | |
|---|---|
| Gaschromatograph | Agilent 7820A |
| Säule | HP-1, 100m |
| Säulendurchmesser | 0,25 mm |
| Filmdicke | 0,5 $\mu$m |
| Trägergas | Wasserstoff 3.0 |
| Splitverhältnis | 1:100 |
| Detektor | Flammenionisationsdetektor |
| Druck Probenschleife | 800 mbar |
| Temperatur Probenschleife | 200°C |

Unter Berücksichtigung der zuvor bestimmten Responsefaktoren und Retentionszeiten wurden die durch den Detektor ermittelten Peakflächen den entsprechenden Substanzen zugeordnet und in Massen bzw. Stoffmengen umgerechnet.

Durch den komplexen Reaktionsmechanismus sind die Stöchiometriefaktoren der ablaufenden Reaktionen nicht immer bekannt und somit wurde zur Berechnung der Selektivitäten und Umsätze die Stoffmenge Kohlenstoff in jeder detektierten Substanz $n_{c,i}$ verwendet. Dieser ergibt sich aus dem Produkt der berechneten Stoffmenge der Substanz i im Produktgemisch und der Anzahl Kohlenstoffatome je Molekül $C_i$:

$$n_{C,i} = n_i \cdot C_i$$

$$n_{C,gesamt} = \Sigma\, n_{C,i}$$

Mit dieser Konvention ergibt sich für den Umsatz und die Selektivitäten:

$$X = \frac{n_{C,gesamt} - n_{C,Ethanol}}{n_{C,gesamt}}$$

$$S_i = \frac{n_{C,i}}{n_{C,gesamt} - n_{C,\,Ethanol}}$$

**Tabelle 1**

| Bsp | Katalysator | WHSV | T | p, abs | Umsatz | Slectivität Butadien | Selectivität Ethen | Ausbeute Butadien | Butadiendurchsatz |
|---|---|---|---|---|---|---|---|---|---|
| | [mol-%] | [1/h] | [°C] | [bar] | [%] | [%] | [%] | [%] | [g/g*h] |
| 1 | MgO-HDK 85:15 | 1,3 | 450 | 5,5 | 40,2 | 45,4 | 41,7 | 18,3 | 0,14 |
| 2 | MgO-CaO-HDK 84,9:0,1:15 | 1,3 | 450 | 5,5 | 46,2 | 51,1 | 15,3 | 23,6 | 0,18 |
| 3 | MgO-CaO-HDK 82,5:2,5:15 | 1,7 | 450 | 5,5 | 47,0 | 54,4 | 22,4 | 25,6 | 0,26 |
| 4 | MgO-CaO-HDK 80:5:15 | 1,4 | 450 | 5,5 | 42,4 | 53,1 | 18,3 | 22,5 | 0,18 |
| WHSV = Katalysatorbelastung (Weight-Hour-Space-Velocity; Ethanolzufuhr in g/h pro g Katalysator) | | | | | | | | | |

Entionisiertes Wasser im Sinne der vorliegenden Erfindung ist ein durch die Entfernung aller austauschbaren Ionen hergestelltes Wasser mit einer elektrischen Leitfähigkeit <10μS/cm.

**Patentansprüche**

1. Katalysator, enthaltend

   (I) wenigstens eine Sauerstoffverbindung des Magnesiums und wenigstens eine Sauerstoffverbindung des Siliciums und
   (II) Calciumoxid.

2. Katalysator nach Anspruch 1, **dadurch gekennzeichnet, dass** dieser

   (I) 90 bis 99,9 Mol.-% wenigstens einer Sauerstoffverbindung des Magnesiums und wenigstens einer Sauerstoffverbindung des Siliciums und
   (II) 0,1 bis 10 Mol.-% Calciumoxid enthält, mit der Maßgabe, dass die Summe aller Molprozente stets 100 ergibt.

3. Katalysator gemäß einem der Ansprüche 1 oder 2, **dadurch gekennzeichnet, dass** in der Komponente (I) das molare Verhältnis von Magnesium zu Silicium im Bereich von 3,5:1 bis 15:1, vorzugsweise im Bereich von 4:1 bis 15 :1 und besonders bevorzugt im Bereich von 5,5:1 bis 12,5:1 liegt.

4. Katalysator gemäß einem der Ansprüche 1 bis 3, **dadurch gekennzeichnet, dass** die Sauerstoffverbindungen des Magnesiums ausgewählt sind aus der Gruppe Magnesiumhydroxid und Magnesiumoxid, wobei Magnesiumoxid bevorzugt ist.

5. Katalysator gemäß einem der Ansprüche 1 bis 4, **dadurch gekennzeichnet, dass** die Sauerstoffverbindungen des Siliciums ausgewählt sind aus der Gruppe der kristallinen oder nicht-kristallinen Modifikationen bzw. Erscheinungsformen des Siliciumdioxids und Kieselsäuren der Formel $H_{2n+2}Si_nO_{3n+1}$ wobei n eine natürliche Zahl ist.

6. Katalysator gemäß Anspruch 5, **dadurch gekennzeichnet, dass** er als Sauerstoffverbindungen des Siliciums pyrogene Kieselsäuren enthält.

7. Katalysator gemäß einem der Ansprüche 1 bis 6, **dadurch gekennzeichnet, dass** er als Sauerstoffverbindungen des Magnesiums und Siliciums Magnesiumsilikate, bevorzugt Talk und Sepiolith enthält.

8. Katalysator gemäß einem der Ansprüche 1 bis 7, **dadurch gekennzeichnet, dass** Komponente (I) für wenigstens MgO und wenigstens $SiO_2$ und Komponente (II) für CaO steht.

9. Katalysator gemäß einem der Ansprüche 1 bis 8, **dadurch gekennzeichnet, dass** er weiterhin zumindest eine Übergangsmetallverbindung enthält, wobei die Übergangsmetallverbindungen vorzugsweise ausgewählt sind aus Sauerstoff-Verbindungen der Elemente Titan, Vanadium, Molybdän, Wolfram, Mangan, Kupfer, Nickel, Zink, oder Chrom.

10. Katalysator gemäß Anspruch 9, **dadurch gekennzeichnet, dass** das molare Verhältnis von Magnesium zu Übergangsmetall von 15:1 bis 1000:1 beträgt.

11. Katalysator gemäß einem der Ansprüche 1 bis 10, **dadurch gekennzeichnet, dass** dieser 0,1 Gew.-% oder weniger an Alkalimetallverbindungen berechnet auf $M_2O$ enthält, wobei M ein Alkalimetall, wie insbesondere Lithium, Natrium oder Kalium ist, vorzugsweise weniger als 0,08 Gew.-%.

12. Katalysator gemäß einem der Ansprüche 1 bis 11, **dadurch gekennzeichnet, dass** deren spezifische Oberfläche 20 bis 500 $m^2$/g beträgt.

13. Verfahren zur Herstellung von Katalysatoren gemäß einem der Ansprüche 1 bis 12, **dadurch gekennzeichnet, dass** es zumindest folgende Schritte umfasst:

   A) Herstellung eines Katalysatormaterials durch

ia) Fällung zumindest einer im Wesentlichen wasserlöslichen Magnesiumverbindung auf zumindest eine feste Sauerstoffverbindung des Siliciums und Calciumoxid, oder

ib) Fällung zumindest einer im Wesentlichen wasserlöslichen Calciumverbindung auf zumindest eine feste Sauerstoffverbindung des Siliciums und auf zumindest eine feste Sauerstoffverbindung des Magnesiums, oder

ic) Fällung zumindest einer nicht festen Siliciumverbindung auf zumindest eine feste Sauerstoffverbindung des Magnesiums und Calciumoxid, oder

id) Fällung zumindest einer nicht festen Siliciumverbindung und anschließend einer wasserlöslichen Calciumverbindung auf zumindest eine feste Sauerstoffverbindung des Magnesiums, oder

ie) Fällung zumindest einer nicht festen Siliciumverbindung und anschließend einer wasserlöslichen Magnesiumverbindung auf Calciumoxid, oder

if) Fällung zumindest einer löslichen Magnesiumverbindung und zumindest einer löslichen Calciumverbindung auf zumindest eine feste Sauerstoffverbindung des Siliciums,

jeweils in einem wässrigen Reaktionsmedium und Abtrennung des so erhaltenen, gefällten Katalysatormaterials vom wässrigen Reaktionsmedium,

ii) Mischen von Sauerstoffverbindungen des Magnesiums oder Magnesiumcarbonat, Calciumoxid und Sauerstoffverbindungen des Siliciums als Katalysatormaterial,

B) gegebenenfalls Vermahlung des Katalysatormaterials,

C) gegebenenfalls Herstellung von Formkörpern enthaltend das gemäß Schritt A) und/oder B) erhaltene Katalysatormaterial,

D) Kalzinierung des Katalysatormaterials oder der Formkörper.

14. Verfahren zur katalytischen Herstellung von 1,3-Butadien, **dadurch gekennzeichnet, dass** es in Gegenwart wenigstens eines Katalysators gemäß einem der Ansprüche 1 bis 12 durchgeführt wird.

15. Verfahren nach Anspruch 14, **dadurch gekennzeichnet**, das ein Ethanol enthaltender Gasstrom mit dem Katalysator in Kontakt gebracht wird, bevorzugt bei einer Temperatur im Bereich von 200°C bis 500°C.

Fig. 1

Europäisches
Patentamt

European
Patent Office

Office européen
des brevets

# EUROPÄISCHER RECHERCHENBERICHT

Nummer der Anmeldung

EP 14 15 9488

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | RU 2 503 650 C1 (NII JARSINTEZ OAO NII JARSINTEZ AOOT [RU]) 10. Januar 2014 (2014-01-10) * Zusammenfassung * ----- | 1-15 | INV. B01J23/02 C07C1/20 C07C11/167 B01J37/02 |
| X | RU 2 459 788 C2 (FEDERAL NOE G BJUDZHETNOE UCHREZHDENIE NAUKI INST NEFTEKHIMICHESKOGO S) 27. August 2012 (2012-08-27) * Zusammenfassung * ----- | 1-15 | |
| A | GB 573 631 A (WACLAW SZUKIEWICZ) 29. November 1945 (1945-11-29) * das ganze Dokument * ----- | 1-15 | |
| E | EP 2 712 673 A1 (LANXESS DEUTSCHLAND GMBH [DE]) 2. April 2014 (2014-04-02) * das ganze Dokument * ----- | 1-15 | |
| | | | RECHERCHIERTE SACHGEBIETE (IPC) |
| | | | B01J C07C |

Der vorliegende Recherchenbericht wurde für alle Patentansprüche erstellt

| Recherchenort | Abschlußdatum der Recherche | Prüfer |
|---|---|---|
| Den Haag | 3. Juli 2014 | Schoofs, Bart |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

....................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 14 15 9488

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

03-07-2014

| Im Recherchenbericht angeführtes Patentdokument | | Datum der Veröffentlichung | Mitglied(er) der Patentfamilie | | Datum der Veröffentlichung |
|---|---|---|---|---|---|
| RU 2503650 | C1 | 10-01-2014 | --------- | | |
| RU 2459788 | C2 | 27-08-2012 | KEINE | | |
| GB 573631 | A | 29-11-1945 | KEINE | | |
| EP 2712673 | A1 | 02-04-2014 | EP | 2712673 A1 | 02-04-2014 |
| | | | WO | 2014049158 A1 | 03-04-2014 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- GB 331482 A

- WO 2012015340 A1

**In der Beschreibung aufgeführte Nicht-Patentliteratur**

- **H. NIIYAMA et al.** *Bulletin of the Chemical Society of Japan,* 1972, vol. 45, 655-659